(19) Europäisches Patentamt
European Patent Office
Office européen des brevets

(11) EP 4 759 864 A1

(12) EUROPEAN PATENT APPLICATION
published in accordance with Art. 153(4) EPC

(43) Date of publication:
**17.06.2026 Bulletin 2026/25**

(21) Application number: **24851382.2**

(22) Date of filing: **10.06.2024**

(51) International Patent Classification (IPC):
***C08G 77/38*** (2006.01) ***A61L 27/00*** (2006.01)
***A61L 27/18*** (2006.01) ***C08F 290/06*** (2006.01)

(52) Cooperative Patent Classification (CPC):
**A61L 27/00; A61L 27/18; C08F 290/06; C08G 77/38**

(86) International application number:
**PCT/JP2024/020976**

(87) International publication number:
**WO 2025/032949 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.08.2023 JP 2023128873**

(71) Applicant: **SHIN-ETSU CHEMICAL CO., LTD.**
**Tokyo 100-0005 (JP)**

(72) Inventor: **OKAMURA, Kaoru**
**Annaka-shi, Gunma 379-0224 (JP)**

(74) Representative: **Schicker, Silvia**
**Wuesthoff & Wuesthoff**
**Patentanwälte und Rechtsanwalt PartG mbB**
**Schweigerstraße 2**
**81541 München (DE)**

# (54) POLYSILOXANE COMPOUND, POLYMER, HYDROGEL, MEDICAL MATERIAL, AND METHOD FOR PRODUCING POLYSILOXANE COMPOUND

(57) The present invention is a polysiloxane compound including one or more (meth)acryloxy groups per molecule, wherein the polysiloxane compound has a tertiary hydroxyl group at the terminal of a side chain. This can provide: a polysiloxane compound suitable for medical materials; a polymer using the polysiloxane compound; a hydrogel containing the polymer; a medical material containing the polymer; and a method for manufacturing the polysiloxane compound.



Processed by Luminess, 75001 PARIS (FR)

## Description

TECHNICAL FIELD

The present invention relates to a polysiloxane compound, a polymer, a hydrogel, a medical material, and a method for manufacturing the polysiloxane compound.

BACKGROUND ART

Conventionally, monomers with siloxane have been known as a compound used for medical materials such as ophthalmic devices. For example, 3-[tris(trimethylsiloxy)silyl]propyl methacrylate (TRIS) is widely used as a monomer for ophthalmic devices. Polymers obtained by copolymerization of the TRIS and N,N-dimethylacrylamide or N-vinyl-2-pyrrolidone being a hydrophilic monomers have the beneficial feature of high oxygen permeability. However, highly hydrophobic siloxane monomers are not highly compatible with these hydrophilic monomers, and it causes a problem resulting in phase separation and cloudiness when the hydrogel is prepared to be used as a medical material.
Patent Document 1 discloses a siloxane having a primary or secondary hydroxyl group, represented by the following formulae (a) and (a').

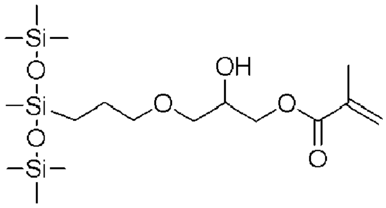 (a) 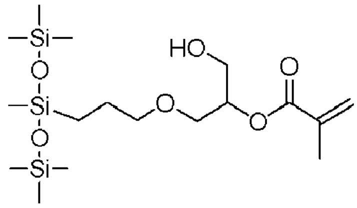 (a')

Further, Patent Document 2 discloses a siloxane having a primary or secondary hydroxyl group in its side chain, as represented by the following formula (b).

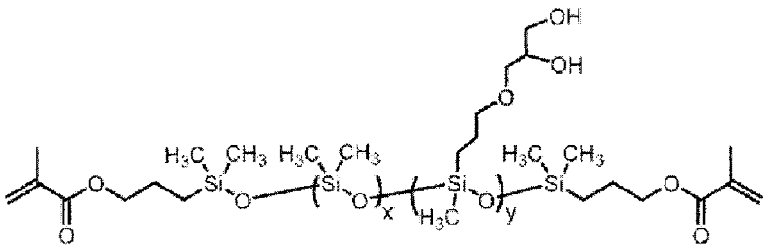 x=122.5; y=11.0 (b)

These compounds exhibit good hydrophilicity because they have a hydroxyl group in their molecules. Therefore, it has the advantage of excellent compatibility with a hydrophilic monomer.
However, when producing a hydrogel with the above compound, which contains a (poly)siloxane and a primary or secondary hydroxyl group, these highly reactive hydroxyl groups may cause undesirable results. For example, there is a risk that a radical may be added to the above hydroxyl group, forming a crosslinked structure due to hydroxyl radicals. This causes a defect in appearance, an unexpected increase in hardness, or an unexpected decrease in flexibility. Therefore, conventional siloxane compounds cannot provide medical materials having useful transparency, hydrophilic properties, or sufficient strength. Thus, there remains still a demand for compounds and compositions that can overcome such drawbacks.

CITATION LIST

PATENT LITERATURE

Patent Document 1: JP2005-170827A
Patent Document 2: JP2021-073346A

SUMMARY OF INVENTION

TECHNICAL PROBLEM

An object of the present invention is to provide: a polysiloxane compound suitable for medical materials; a polymer using the polysiloxane compound; a hydrogel containing the polymer; a medical material containing the polymer; and a method for manufacturing the polysiloxane compound.

SOLUTION TO PROBLEM

To solve the above problems, the present invention provides a polysiloxane compound comprising one or more (meth)acryloxy groups per molecule, wherein the polysiloxane compound has a tertiary hydroxyl group at the terminal of a side chain.
Such a polysiloxane compound can be used suitably for medical materials.
In the present invention, the polysiloxane compound is preferably represented by the following general formula (1),

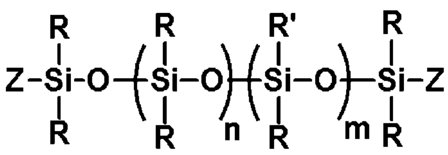

(1)

wherein "Z" each independently represents an organic group having 1 to 20 carbon atoms; "R" each independently represents a monovalent hydrocarbon group having 1 to 6 carbon atoms; "R'" each independently represents a monovalent hydrocarbon group having a tertiary hydroxyl group at the terminal, having 3 to 20 carbon atoms, and optionally having an ether bond at the non-terminal of the group; "n" represents an integer of 0 to 200; "m" represents an integer of 3 to 200; and one or more of "Z"s are a (meth)acryloxy group.
Such a polysiloxane compound can be more suitably used as a medical material.
Further, in the present invention, in the general formula (1), "Z" is preferably represented by the following general formula (2),

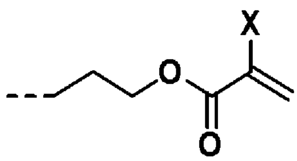

(2)

wherein "X" represents a hydrogen atom or a methyl group, and a broken line represents an attachment point to a silicon atom.
Such a polysiloxane compound can be further more suitably used as a medical material.
Further, in the present invention, in the general formula (1), "R" preferably is a methyl group.
The inventive polysiloxane is preferably such a compound.
Further, in the present invention, in the general formula (1), "R'" is preferably represented by the following general formula (3) or (4),

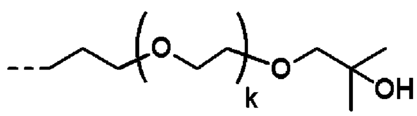

(3)

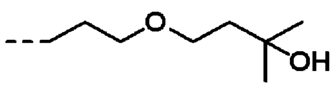

(4)

wherein "k", in the general formula (3), represents an integer of 0 to 5, and a broken line, in the general formulae (3) and (4), represents an attachment point to a silicon atom.
The inventive polysiloxane is more preferably such a compound.
In this event, in the general formula (3), "k" preferably is 0 or 1.
Such a polysiloxane compound can be further more suitably used as a medical material.

Further, in the present invention, in the general formula (1), it is preferable that "n" is an integer of 0 to 100, "m" is an integer of 3 to 100, and m/(n+m) is 0.15 or more.

Such a polysiloxane compound can be further more suitably used as a medical material.

In this event, in the general formula (1), m/(n+m) is preferably 0.3 or more.

Such a polysiloxane compound can be further more suitably used as a medical material.

Further, the present invention provides a polymer being a polymerized product of the polysiloxane compound described above.

Such a polymer has good compatibility with other compounds having a polymerizable group such as a (meth) acryloxy group.

In this event, the mass ratio of the polysiloxane compound is preferably 20 mass% or more relative to the total mass of the polymer.

Such a polymer is likely to exhibit the properties of the polysiloxane compound.

Further, the present invention provides a hydrogel or a medical material including the polymer described above.

The inventive polymer can be used suitably for such uses.

Further, the present invention provides a method for manufacturing a polysiloxane compound,

wherein the method for manufacturing the polysiloxane compound comprises a step of obtaining the polysiloxane compound represented by the general formula (1) by a hydrosilylation of a compound having an unsaturated group and a tertiary hydroxyl group at the terminal, and an organohydrogenpolysiloxane compound represented by the following formula (5),

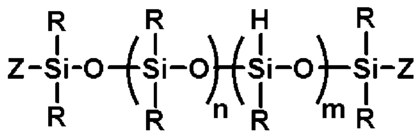

(5)

wherein "Z" each independently represents an organic group having 1 to 20 carbon atoms; "R" each independently represents a monovalent hydrocarbon group having 1 to 6 carbon atoms; "n" represents an integer of 0 to 200; "m" represents an integer of 3 to 200; and one or more of "Z"s are a (meth)acryloxy group,

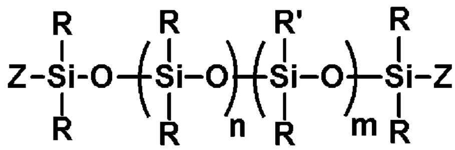

(1)

wherein "R'" each independently represents a monovalent hydrocarbon group having a tertiary hydroxyl group at the terminal, having 3 to 20 carbon atoms, and optionally having an ether bond at the non-terminal of the group; and "Z", "R", "n", and "m" represent the same defined above.

Such a manufacturing method for the polysiloxane compound makes it possible to obtain the polysiloxane compound represented by the general formula (1) with high purity.

In this event, the compound having an unsaturated group and a tertiary hydroxyl group at the terminal is contained in 1.0 to 3.0 molar equivalents relative to a hydrogensiloxy unit.

Such a method of manufacturing the polysiloxane compound makes it possible to obtain the polysiloxane compound with high purity economically.

In this event, the content ratio of the polysiloxane compound represented by the general formula (1) is preferably 90% or more.

The polysiloxane compound with such a content ratio can be further more suitably used as a medical material.

ADVANTAGEOUS EFFECTS OF INVENTION

The inventive polysiloxane compound has excellent compatibility with hydrophilic monomers due to having a tertiary hydroxyl group at the terminal of the side chain. Furthermore, when used as medical materials, it does not cause reduced transparency, an unexpected increase in hardness, or an unexpected decrease in flexibility. In addition, the (co) polymer, having a repeating unit derived from the polymerization of the (meth)acryloxy groups of the inventive polysiloxane compounds, has favorable strength. The inventive polysiloxane compound is useful as a monomer for medical materials.

DESCRIPTION OF EMBODIMENTS

As described above, it has been desired to develop a polysiloxane compound suitable for medical materials; a polymer using the polysiloxane compound; a hydrogel containing the polymer; a medical material containing the polymer; and a method for manufacturing the polysiloxane compound.

As a result of the present inventor’s diligent study to solve the above problems, it is found that a polysiloxane compound having one or more (meth)acryloxy groups per molecule, wherein the polysiloxane compound has a tertiary hydroxyl group at the terminal of a side chain has excellent compatibility with other hydrophilic monomers and that a (co) polymer of the polysiloxane compound and a hydrophilic monomer has excellent flexibility.

That is, the present invention is a polysiloxane compound comprising one or more (meth)acryloxy groups per molecule, wherein the polysiloxane compound has a tertiary hydroxyl group at the terminal of a side chain.

Hereinafter, the present invention is described in detail, but is not limited thereto.

[Polysiloxane Compound]

The present invention is a polysiloxane compound having one or more (meth)acryloxy groups per molecule, and the polysiloxane compound has a tertiary hydroxyl group at the terminal of a side chain.

The compound is featured to have a polysiloxane structure and a tertiary hydroxyl group at the terminal of a side chain of the polysiloxane compound. In the inventive polysiloxane compound, this tertiary hydroxyl group works as a hydrophilic group, which is sterically restricted. It is conjectured that this suppresses a side reaction during the reaction, and that, in polymers ((co)polymers) having a repeating unit derived from the polymerization of the (meth)acryloxy groups of the inventive polysiloxane compounds, resulting in generating a strong hydrogen-bonding property derived from the directional nature of a hydrogen bond to exhibit high strength. That is, the inventive polysiloxane compound is highly compatible with hydrophilic monomers, can provide desirable strength in the derived (co)polymer, and, in addition, does not cause an unexpected increase in hardness or an unexpected decrease in flexibility.

Particularly, the polysiloxane compound is preferably represented by the following general formula (1).

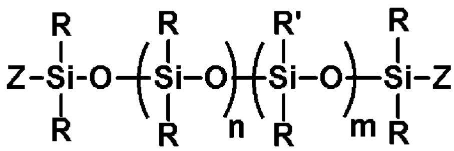

(1)

In the formula, "Z" each independently represents an organic group having 1 to 20 carbon atoms; "R" each independently represents a monovalent hydrocarbon group having 1 to 6 carbon atoms; "R’" each independently represents a monovalent hydrocarbon group having a tertiary hydroxyl group at the terminal, having 3 to 20 carbon atoms, and optionally having an ether bond at the non-terminal of the group; "n" represents an integer of 0 to 200; "m" represents an integer of 3 to 200; and one or more of "Z"s are a (meth)acryloxy group.

In the above general formula (1), "Z" each independently represents an organic group having 1 to 20 carbon atoms, and is preferably represented by the following general formula (2).

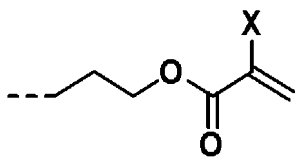

(2)

In the formula, "X" represents a hydrogen atom or a methyl group, and a broken line represents an attachment point to a silicon atom.

In the above general formula (1), "R" each independently represents a monovalent hydrocarbon group having 1 to 6 carbon atoms. The monovalent hydrocarbon groups include: alkyl groups, such as a methyl group, an ethyl group, a propyl group, an n-butyl group, a t-butyl group, a pentyl group, and a hexyl group; cycloalkyl groups, such as a cyclopentyl group and a cyclohexyl group; aryl groups, such as a phenyl group. "R" is preferably an alkyl group having 1 to 4 carbon atoms, more preferably a methyl group.

In the above general formula (1), "R’" each independently represents a monovalent hydrocarbon group having a tertiary hydroxyl group at the terminal, having 3 to 20 carbon atoms, and optionally having an ether bond at the non-terminal of the group. "R’" is preferably represented by the following general formula (3) or (4).

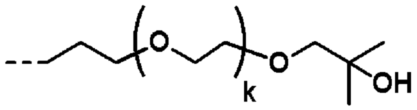

(3)

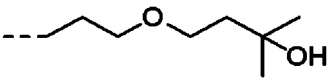

(4)

In the general formula (3), "k" represents an integer of 0 to 5, and, in the general formulae (3) and (4), a broken line represents an attachment point to a silicon atom.

In the above general formula (3), "k" represents an integer of 0 to 5, preferably 0 or 1.

In the above general formula (1), "n" represents an integer of 0 to 200, and "m" represents an integer of 3 to 200. It is preferable that "n" is an integer of 0 to 100, "m" is an integer of 3 to 100, and m/(n+m) is 0.15 or more. m/(n+m) is more preferably 0.3 or more, and further preferably 0.3 to 1.0.

[Method for Manufacturing Polysiloxane Compound]

Hereinafter, a method for manufacturing the polysiloxane compound represented by the above general formula (1) will be described.

The present invention provides a method for manufacturing a polysiloxane compound,

wherein the method for manufacturing the polysiloxane compound comprises a step of obtaining the polysiloxane compound represented by the general formula (1) by a hydrosilylation of a compound having an unsaturated group and a tertiary hydroxyl group at the terminal, and an organohydrogenpolysiloxane compound represented by the following general formula (5),

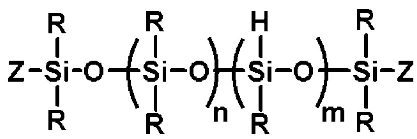

(5)

wherein "Z" each independently represents an organic group having 1 to 20 carbon atoms; "R" each independently represents a monovalent hydrocarbon group having 1 to 6 carbon atoms; "n" represents an integer of 0 to 200; "m" represents an integer of 3 to 200; and one or more of "Z"s are a (meth)acryloxy group.

The inventive method for manufacturing the polysiloxane compound features the use of a compound having an unsaturated group and a tertiary hydroxyl group at the terminal as a material. In the inventive method for manufacturing the polysiloxane compound, because the hydroxyl group is a tertiary hydroxyl group, an undesirable side reaction caused by the hydroxyl group during the hydrosilylation reaction can be suppressed. Thereby, it is possible to obtain the inventive polysiloxane compound with high purity.

The above hydrosilylation reaction may be performed according to a conventionally publicly known method. In the present invention, the organohydrogensiloxane compound represented by the above general formula (5) may be reacted by adding the compound having an unsaturated group and a tertiary hydroxyl group at the terminal, for example, in an amount of 1 molar equivalent or more. The reaction temperature is not particularly limited, but is preferably around a level not exceeding the boiling point of the solvent used. For example, it is preferable to perform at a temperature of about 0°C to about 150°C. The hydrosilylation reaction may be performed in the presence of a solvent, a hydrosilylation catalyst, or a stabilizer. The solvent, the hydrosilylation catalyst, and the stabilizer may be conventionally publicly known and are not particularly limited.

In the above hydrosilylation reaction, the compound having an unsaturated group and a tertiary hydroxyl group at the terminal is preferably added in an amount of 1 molar equivalent or more relative to a hydrogensiloxy unit, more preferably 1.0 to 3.0 molar equivalents, further preferably 1.2 to 2.0 molar equivalents, particularly preferably 1.5 to 2.0 molar equivalents. By adding 1 molar equivalent or more of the compound having an unsaturated group and a tertiary hydroxyl group at the terminal to the hydrogensiloxy unit, the remaining of the hydrogensiloxy unit and side reactions can be suppressed. Although there is no upper limit, it is preferable to add 3.0 molar equivalents or less from the viewpoint of yield and cost efficiency.

(Hydrosilylation Catalyst)

The hydrosilylation catalysts are preferably, for example, noble metal catalysts, especially platinum catalysts derived from chloroplatinic acid. In particular, it is preferably a catalyst in which the hydrogen ion of the chloroplatinic acid is completely neutralized by sodium bicarbonate to improve the stability of the platinum catalyst. For example, a complex (Karstedt catalyst) of chloroplatinic acid neutralized by the sodium bicarbonate with 1,1,3,3-tetramethyl-1,3-divinyldisiloxane is more preferable.

The amount of the hydrosilylation catalyst added may be sufficient in a catalytic amount to proceed the hydrosilylation reaction. For example, a complex of chloroplatinic acid neutralized by the sodium bicarbonate with 1,1,3,3-tetramethyl-1,3-divinyldisiloxane may be used in an amount of 1 ppm to 80 ppm in terms of platinum, relative to the mass of the organohydrogensiloxane compound represented by the above general formula (5).

(Solvent)

Examples of solvents include: glycol ethers solvents, such as methyl cellosolve, ethyl cellosolve, isopropyl cellosolve, butyl cellosolve, propylene glycol monomethyl ether, diethylene glycol monomethyl ether, triethylene glycol monomethyl ether, polyethylene glycol monomethyl ether, ethylene glycol dimethyl ether, diethylene glycol dimethyl ether, triethylene glycol dimethyl ether, and polyethylene glycol dimethyl ether; ester solvents, such as ethyl acetate, butyl acetate, amyl acetate, ethyl lactate, and methyl benzoate; aliphatic hydrocarbon solvents, such as linear hexane, linear heptane, and linear octane; alicyclic hydrocarbon solvents such as cyclohexane and ethylcyclohexane; ketone solvents, such as acetone, methyl ethyl ketone, and methyl isobutyl ketone; aromatic hydrocarbon solvents, such as benzene, toluene, and xylene; petroleum solvents; and alcoholic solvents, such as methyl alcohol, ethyl alcohol, linear propyl alcohol, isopropyl alcohol, linear butyl alcohol, isobutyl alcohol, tert-butyl alcohol, ethylene glycol, diethylene glycol, triethylene glycol, tetraethylene glycol, and polyethylene glycol. One kind of the above solvents may be used, or two or more kinds thereof may be used in combination.

(Stabilizer)

Examples of the stabilizer include phenolic antioxidants, phosphorus antioxidants, amine antioxidants, and sulfur-based antioxidants. Examples of the phenolic antioxidants include, but are not particularly limited to, a compound or the like selected from the group consisting of p-methoxyphenol, di-tert-butyl-p-cresol, pyrogallol, tert-butylcatechol, 4,4-thiobis(3-methyl-6-tert-butylphenol), 2,2'-methylenebis(4-methyl-6-t-butylphenol), phenolic resins, and cresol resins. Examples of the phosphorous antioxidants include, but are not particularly limited to, tris[2-[[2,4,8,10-tetrakis(1,1-dimethylethyl)dibenzo[d,f][1,3,2]dioxaphosphepin-6-yl]oxy]ethyl]amine, tris[2-[(4,6,9,11-tetra-tert-butyldibenzo[d,f][1,3,2]dioxaphosphepin-2-yl)oxy]ethyl]amine, and ethylbis(2,4-di-tert-butyl-6-methylphenyl) phosphite. Examples of the amine antioxidant include, but are not particularly limited to, tri- or tetra-C1-3 alkylpiperidine or derivatives thereof, bis(2,2,6,6-tetramethyl-4-piperidyl)oxalate, 1,2-bis(2,2,6,6-tetramethyl-4-piperidyloxy)ethane, phenylnaphthylamine, N,N'-diphenyl-1,4-phenylenediamine, and N-phenyl-N'-cyclohexyl-1,4-phenylenediamine. Examples of the sulfur-based antioxidant include, but are not particularly limited to, dilaurylthiodipropionate and distearylthiodipropionate. One kind of the stabilizers may be used, or two or more kinds thereof may be used in combination.

In the above hydrosilylation reaction, the end point of the reaction can be confirmed according to a conventionally publicly known method. For example, the end point can be confirmed by the disappearance of the peak of the material compound using $^1$H-NMR analysis, thin layer chromatography (TLC), high performance liquid chromatography (HPLC), gas chromatography (GC), etc.

After the reaction is completed, the resultant product may be purified according to conventionally publicly known methods. For example, the resultant product can be isolated by washing the organic layer with water, then removing the solvent. Alternatively, it is possible to use reduced-pressure distillation, activated carbon treatment, etc.

For an example of the method for manufacturing the inventive polysiloxane compound, 1 molar equivalent of the organohydrogensiloxane compound represented by the above general formula (5), 1.5 molar equivalents of the compound having an unsaturated group and a tertiary hydroxyl group at the terminal, and 10 ppm of, in terms of platinum based on the mass of the organohydrogensiloxane compound, toluene solution (platinum content 0.5 wt%) of a complex of a neutralized product of chloroplatinic acid by sodium bicarbonate and vinylsiloxane are mixed and subjected to heat agitation at 80°C. The reaction is completed in about 6 hours. The progress of the reaction can be confirmed by monitoring the compound having an unsaturated group and a tertiary hydroxyl group at the terminal or generated compounds with $^1$H-NMR analysis, etc. After completion of the reaction, the unreacted material is distilled off under reduced pressure, and then a polysiloxane compound represented by the above general formula (1) can be obtained.

[Purity of Polysiloxane Compounds]

The purity (content) of the inventive polysiloxane compounds can be confirmed, for example, by performing gel permeation chromatography (GPC) measurements. The measurements may be performed according to conventionally publicly known methods and are not particularly limited, but it is preferable to use an eluent that dissolves the polysiloxane compounds of the present invention. Furthermore, a conventionally publicly known detector, such as a refractive index (RI) detector, can be used. The purity of the polysiloxane compound can be calculated from the area ratio of the target substance on the GPC chromatogram. In this case, it is preferable that the content ratio of the polysiloxane compound is 90% or higher, facilitating the exhibition of the properties of the inventive polysiloxane compound.

[Polymer]

The present invention provides a polymer being a polymerized product of the polysiloxane compound described above. The inventive polysiloxane compound can provide a polymer having a repeating unit derived from (addition) polymerization of a (meth)acryloxy group of the polysiloxane compound. The inventive polysiloxane compound has good compatibility with other compounds (hereinafter, referred to as the polymerizable monomers or the hydrophilic monomers) having a polymerizable group, such as a (meth)acryloxy group. Therefore, a colorless and transparent copolymer can be obtained by copolymerizing with a polymerizable monomer. The polymerization can also be performed by using a single polysiloxane compound alone.

As for the blending ratio of the inventive polysiloxane compound in manufacturing the copolymer having a repeating unit derived from polymerization of the inventive polysiloxane compound and the other copolymerizable (hydrophilic) monomer, the mass ratio of the repeating unit derived from the inventive polysiloxane compound is preferably 20 mass% or more based on the total mass of the polymer. That is, the mass ratio of the polysiloxane compound is preferably 20 mass% or more relative to the total mass of the polymer. More in detail, a repeating unit derived from the (addition) polymerization of the (meth)acryloxy group of the inventive polysiloxane compound is contained in an amount of preferably 20 to 80 parts by mass, more preferably 30 to 60 parts by mass, based on 100 parts by total mass of the inventive polysiloxane compound and the polymerizable (hydrophilic) monomers combined. When the above repeating unit is contained in 20 mass% or more, the properties of the inventive polysiloxane compound are likely to be exhibited.

Examples of the polymerizable monomers include: acrylic monomers; such as (meth)acrylic acid, methyl (meth) acrylate, ethyl (meth)acrylate, (poly)ethylene glycol dimethacrylate, polyalkylene glycol mono(meth)acrylate, polyalkylene glycol monoalkyl ether (meth)acrylate, trifluoroethyl (meth)acrylate, 2-hydroxyethyl (meth)acrylate, and 2,3-dihydroxypropyl (meth)acrylate; acrylic acid derivative, such as N,N-dimethylacrylamide, N,N-diethylacrylamide, N-acryloylmorpholine, and N-methyl(meth)acrylamide; N-vinylpyrrolidone, etc., other unsaturated aliphatic or aromatic compounds, such as crotonic acid, cinnamic acid, and vinylbenzoic acid; and siloxane monomer having a polymerizable group, such as a(math)acrylic group. One kind of these may be used, or two or more kinds thereof may be used in combination.

The copolymerization of the inventive polysiloxane compound and the other polymerizable monomers described above may be performed according to a conventionally publicly known method. For example, known polymerization initiators, such as thermal polymerization initiators or photopolymerization initiators, can be used. Examples of the polymerization initiators include 2-hydroxy-2-methyl-1-phenyl-propan-1-one, azobisisobutyronitrile, azobisdimethylvaleronitrile, benzoyl peroxide, tert-butyl hydroperoxide, cumene hydroperoxide, and 2,2'-azobis(2-methylpropionamidine) dihydrochloride. One of these polymerization initiators can be used, or two or more kinds thereof can be used in a mixture. The amount of the polymerization initiator to be blended is preferably 0.001 to 2 parts by mass, more preferably 0.01 to 1 part by mass, based on 100 parts by mass of the total polymerization components.

The polymer having a repeating unit derived from polymerization of a (meth)acryloxy group of the inventive polysiloxane compound has excellent hydrophilicity. Furthermore, the hydrogel obtained from the polymer described above has high transparency and strength. Furthermore, a medical material can also be obtained from the polymer. Therefore, the inventive polysiloxane compounds are suitable for manufacturing medical materials, such as ophthalmic devices, contact lenses, intraocular lenses, and artificial corneas. The method for manufacturing medical materials using the above polymer is not particularly limited, and any conventionally publicly known method for manufacturing medical materials may be followed. For example, when forming a lens shape, such as a contact lens and an intraocular lens, it is possible to use a cutting and processing method, a molding method, etc.

EXAMPLE

Hereinafter, the present invention will be described in detail with reference to Synthesis Examples, Examples, and Comparative Examples. However, the present invention is not limited thereto.

In the following examples, $^{1}$H-NMR analysis was performed using an ECS400 manufactured by JEOL with heavy

chloroform as the measurement solvent.

Gel permeation chromatography (GPC) measurements were all performed under the following conditions.

Apparatus: Tosoh HLC-8320 (RI detector)
Column: TSK Gel Guard Column Super H-H,
TSK Gel Super H2500,
TSK Gel Super HM-N
Temperature: 40°C
Elution solvent: Tetrahydrofuran (THF)
Flow rate: 0.6 mL/min
Sample concentration: 0.3 mass% (THF solution)
Injection amount: 50 μL

Synthesis Example 1

A 500 mL three-necked flask equipped with a Dimroth condenser and a thermometer was charged with 80.0 g of allyl alcohol, 100.0 g of isobutylene oxide, and 46.6 g of potassium tertiary butoxide, heated to 50°C, and aged for 24 hours. After completion of the reaction, the mixture was washed three times with a 10% aqueous solution of sodium chloride, and unreacted materials, etc., were removed by distillation at an internal temperature of 150°C. Simple distillation was then performed to obtain 134.1 g of an unsaturated compound having a tertiary hydroxyl group represented by the following formula (A).

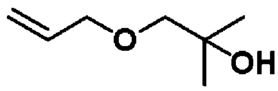

(A)

Synthesis Example 2

The same procedure as in Synthesis Example 1 was performed except that 80.0 g of the allyl alcohol was changed to 141.0 g of allyl glycol to obtain 145.5 g of an unsaturated compound having a tertiary hydroxyl group represented by the following formula (B).

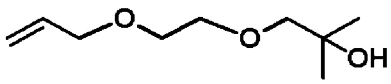

(B)

Synthesis Example 3

A 500 mL three-necked flask equipped with a Dimroth condenser and a thermometer was charged with 100.0 g of 3-methyl-1,3-butanediol, 95.6 g of allyl chloride, 107.6 g of potassium tert-butoxide, and 100.0 g of toluene, heated to 50°C, and aged for 24 hours. After completion of the reaction, the mixture was washed three times with a 10% aqueous solution of sodium chloride, and unreacted materials, etc., were removed by distillation at an internal temperature of 220°C.

Simple distillation was then performed to obtain 113.1 g of an unsaturated compound represented by the following formula (C).

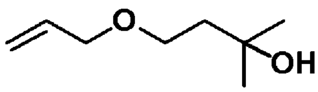

(C)

Example 1-1

A 500 mL three-necked flask equipped with a Dimroth condenser and a thermometer was charged with 50.0 g of a hydrogensiloxane compound represented by the following formula (100), 19.3 g (mole ratio of the unsaturated compound to the hydrogensiloxy unit = 1.5) of an unsaturated compound represented by the above formula (A), 100.0 g of toluene, 0.03 g of a toluene solution (platinum content 0.5 wt%) of a complex of a neutralized product of chloroplatinic acid by sodium bicarbonate and vinylsiloxane, heated to 80°C, and aged for 6 hours. After completion of the reaction, solvents, unreacted materials, etc., were distilled off under reduced pressure at an internal temperature of 100°C, to obtain a colorless, transparent liquid. The yield was 58.8 g. It was confirmed by $^{1}$H-NMR analysis to be a compound represented by

the following formula (100A). GPC measurement was performed, and the area of the target compound was calculated to be 100%.

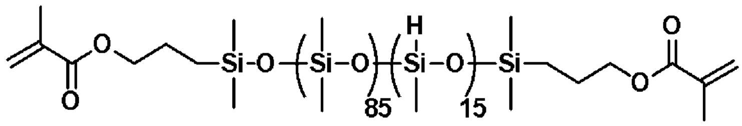

(1 0 0)

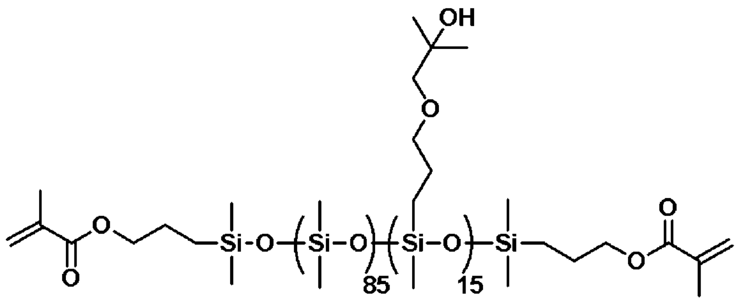

(1 0 0 A)

Example 1-2

The same procedure as in Example 1-1 was performed except that 19.3 g of the unsaturated compound used in Example 1-1, represented by the above formula (A) was changed to 21.4 g (mole ratio of the unsaturated compound to the hydrogensiloxy unit = 1.5) of the unsaturated compound represented by the above formula (C) to obtain a colorless, transparent liquid. The yield was 61.2 g. It was confirmed by $^{1}$H-NMR to be a compound represented by the following formula (100C). GPC measurement was performed, and the area of the target compound was calculated to be 100%.

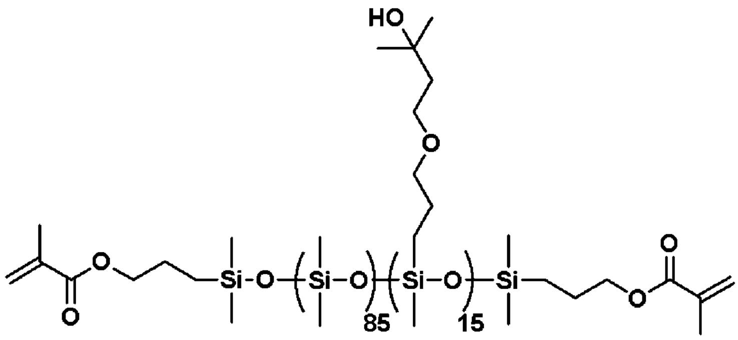

(1 0 0 C)

Example 1-3

The same procedure as in Example 1-1 was performed except that 50.0 g of the hydrogensiloxane compound, used in Example 1-1, represented by the above formula (100) was changed to 50.0 g of the hydrogensiloxane compound represented by the following formula (101) and that 19.3 g of the unsaturated compound represented by the above formula (A) was changed to 95.2 g (mole ratio of the unsaturated compound to the hydrogensiloxy unit = 2.0) of the same to obtain a colorless, transparent solid. The yield was 103.4 g. It was confirmed by $^{1}$H-NMR to be a compound represented by the following formula (101A). GPC measurement was performed, and the area of the target compound was calculated to be 100%.

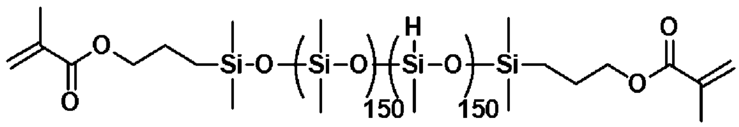

(1 0 1)

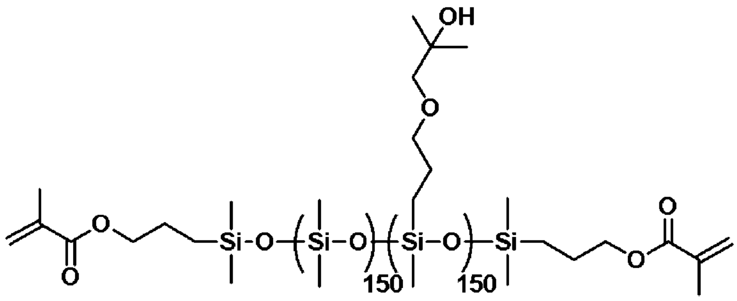

(1 0 1 A)

Example 1-4

The same procedure as in Example 1-1 was performed except that 50.0 g of the hydrogensiloxane compound, used in Example 1-1, represented by the above formula (100) was changed to 20.0 g of the hydrogensiloxane compound represented by the following formula (102) and that 19.3 g of the unsaturated compound represented by the above formula (A) was changed to 99.0 g (mole ratio of the unsaturated compound to the hydrogensiloxy unit = 3.0) of the same to obtain a colorless, transparent solid. The yield was 43.4 g. It was confirmed by $^1$H-NMR to be a compound represented by the following formula (102A). GPC measurement was performed, and the area of the target compound was calculated to be 100%.

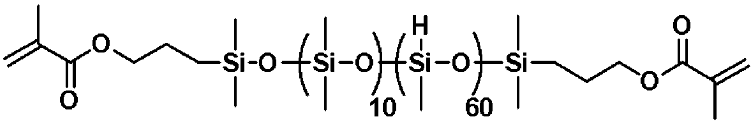

(1 0 2)

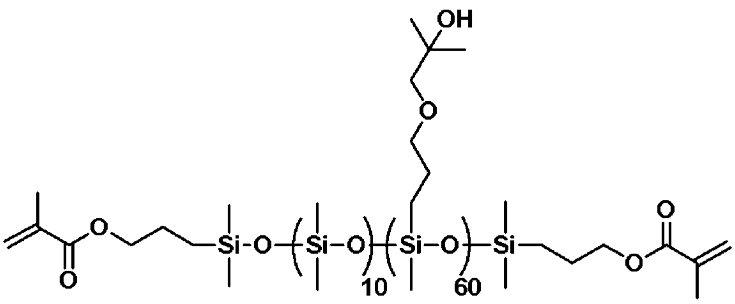

(1 0 2 A)

Example 1-5

The same procedure as in Example 1-1 was performed except that 50.0 g of the hydrogensiloxane compound, used in Example 1-1, represented by the above formula (100) was changed to 20.0 g of the hydrogensiloxane compound represented by the above formula (102) and that 19.3 g of the unsaturated compound represented by the above formula (A) was changed to 88.3 g (mole ratio of the unsaturated compound to the hydrogensiloxy unit = 2.0) of the unsaturated compound represented by the above formula (B) to obtain a colorless, transparent solid. The yield was 50.7 g. It was confirmed by $^1$H-NMR to be a compound represented by the following formula (102B). GPC measurement was performed, and the area of the target compound was calculated to be 100%.

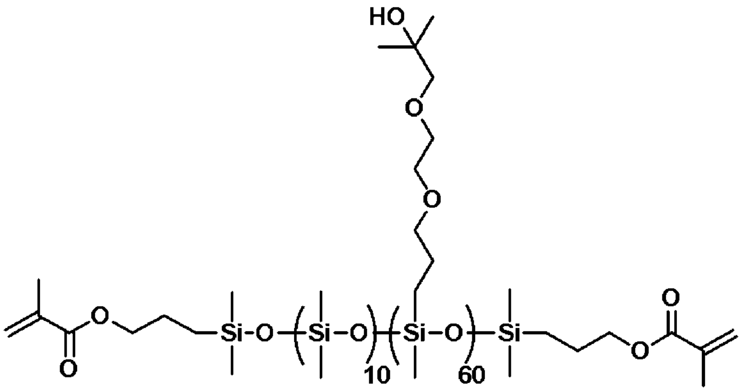

(102B)

Example 1-6

The same procedure as in Example 1-1 was performed except that 50.0 g of the hydrogensiloxane compound, used in Example 1-1, represented by the above formula (100) was changed to 30.0 g of the hydrogensiloxane compound represented by the above formula (103) and that 19.3 g of the unsaturated compound represented by the above formula (A) was changed to 75.4 g (mole ratio of the unsaturated compound to the hydrogensiloxy unit = 2.0) of the same to obtain a colorless, transparent solid. The yield was 60.0 g. It was confirmed by $^1$H-NMR to be a compound represented by the following formula (103A). GPC measurement was performed, and the area of the target compound was calculated to be 100%.

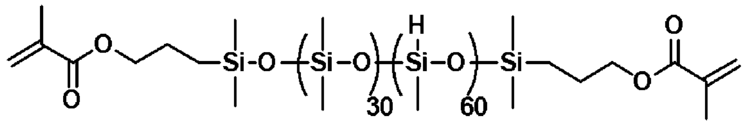

(103)

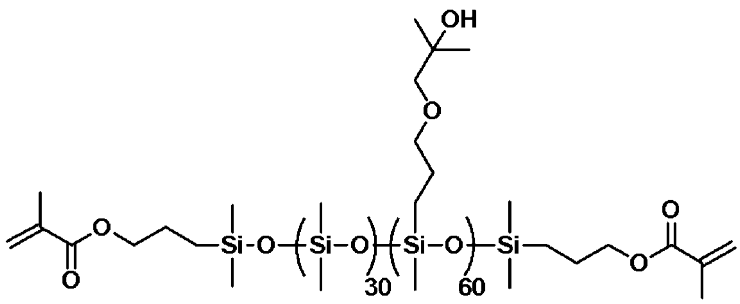

(103A)

Example 1-7

The same procedure as in Example 1-1 was performed except that 50.0 g of the hydrogensiloxane compound, used in Example 1-1, represented by the above formula (100) was changed to 30.0 g of the hydrogensiloxane compound represented by the above formula (103) and that 19.3 g of the unsaturated compound represented by the above formula (A) was changed to 62.6 g (mole ratio of the unsaturated compound to the hydrogensiloxy unit = 1.5) of the unsaturated compound represented by the above formula (C) to obtain a colorless, transparent solid. The yield was 60.2 g. It was confirmed by $^1$H-NMR to be a compound represented by the following formula (103C). GPC measurement was performed, and the area of the target compound was calculated to be 100%.

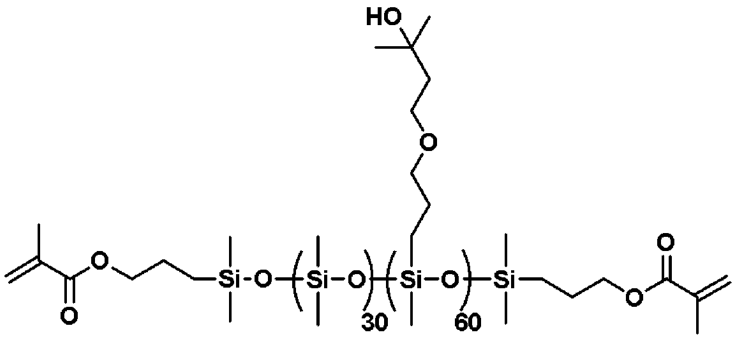

(1 0 3 C)

Example 1-8

The same procedure as in Example 1-1 was performed except that 50.0 g of the hydrogensiloxane compound, used in Example 1-1, represented by the above formula (100) was changed to 30.0 g of the hydrogensiloxane compound represented by the following formula (104) and that 19.3 g of the unsaturated compound represented by the above formula (A) was changed to 45.5 g (mole ratio of the unsaturated compound to the hydrogensiloxy unit = 1.5) of the same to obtain a colorless, transparent solid. The yield was 67.7 g. It was confirmed by $^1$H-NMR to be a compound represented by the following formula (104A). GPC measurement was performed, and the area of the target compound was calculated to be 100%.

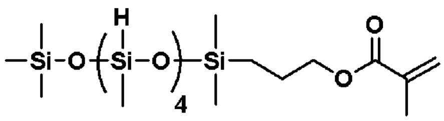

(1 0 4)

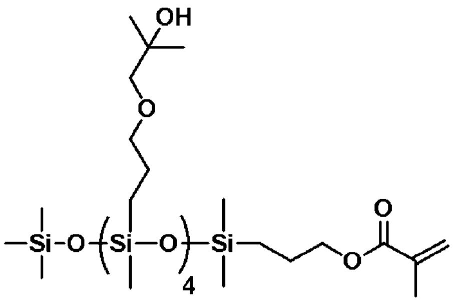

(1 0 4 A)

Comparative Example 1-1

A 500 mL three-necked flask equipped with a Dimroth condenser and a thermometer was charged with 50.0 g of the hydrogensiloxane compound represented by the above formula (100), 19.6 g (mole ratio of the unsaturated compound to the hydrogensiloxy unit = 1.5) of 3-allyloxy-1,2-propanediol, 100.0 g of toluene, and 0.03 g of a toluene solution (platinum content 0.5 wt%) of a complex of a neutralized product of chloroplatinic acid by sodium bicarbonate and vinylsiloxane, heated to 80°C, and aged for 6 hours. After completion of the reaction, solvents were distilled off at an internal temperature of 80°C under reduced pressure, the mixture was added with 50 g of n-hexane, and washed three times with 50 g of acetonitrile. After the washing, solvents were removed by distillation at an internal temperature of 80°C to obtain a cloudy liquid. The yield was 32.2 g. It was confirmed by $^1$H-NMR to be a compound represented by the following formula (200). When GPC measurement was performed, a peak was observed in the high-molecular-weight region, and the area of the target compound was calculated to be 72%.

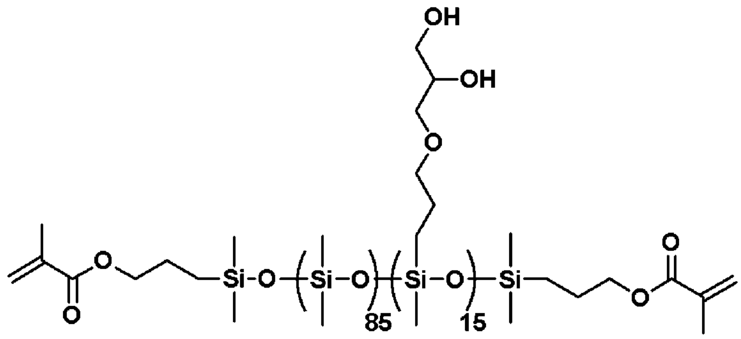

(2 0 0)

Comparative Example 1-2

The same procedure as in Comparative Example 1-1 was performed except that 19.6 g of the 3-allyloxy-1,2-propanediol used in Comparative Example 1-1 was changed to 22.2g (mole ratio of the unsaturated compound to the hydrogensiloxy unit = 2.0) of allyl glycol to obtain a slightly cloudy liquid. The yield was 43.1 g. It was confirmed by $^1$H-NMR to be a compound represented by the following formula (201). When GPC measurement was performed, a peak was observed in the high-molecular-weight region, and the area of the target compound was calculated to be 85%.

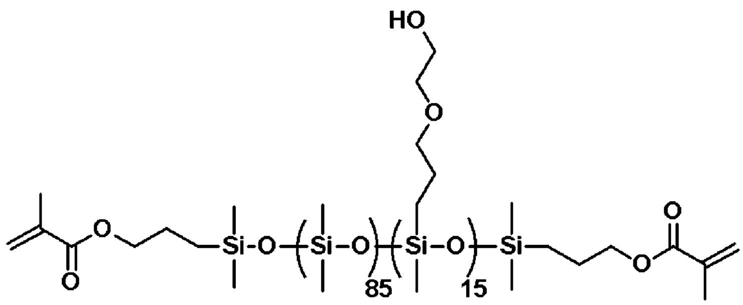

(2 0 1)

Comparative Example 1-3

The same reactions as in Comparative Example 1-1 was proceeded except that 50.0 g of the hydrogensiloxane compound represented by the above formula (100) used in Comparative Example 1-1 was changed to 30.0 g of the hydrogensiloxane compound represented by the above formula (103) and that 19.6 g of 3-allyloxy-1,2-propanediol was changed to 114.8 g (mole ratio of the unsaturated compound to the hydrogensiloxy unit = 3.0) of the same, the mixture gelled during the reaction, failing to form the target product.

Comparative Example 1-4

The same procedure as in Comparative Example 1-1 was performed except that 50.0 g of the hydrogensiloxane compound represented by the above formula (100) used in Comparative Example 1-1 was changed to 30.0 g of the hydrogensiloxane compound represented by the above formula (103) and that 19.6 g of 3-allyloxy-1,2-propanediol was changed to 97.4 g (mole ratio of the unsaturated compound to the hydrogensiloxy unit = 3.0) of allyl glycol to obtain a cloudy gel-like solid. The yield was 21.4 g. Since the obtained compound was partially insoluble in the solvent, only the soluble portion was subjected to GPC measurement. In the GPC measurement, a peak was observed in the high-molecular-weight region, and the area of the target compound was calculated to be 54%.

Comparative Example 1-5

The synthesis was performed in the same procedure except that the hydrogensiloxane compound that is a precursor in the method (Example 6) described in JP2021-073346A was changed to the hydrogensiloxane compound represented by the above formula (100) to obtain a target product (200P) in a colorless, slightly cloudy liquid. When GPC measurement was performed, a peak was observed in the high-molecular-weight region, and the area of the target compound was calculated to be 88%.

As shown in Example 1-1 to 1-8 and Comparative Example 1-1 to 1-5, an unsaturated group-containing compound containing a tertiary hydroxyl group at the terminal suppresses a side reaction accompanied with the

hydrosilylation reaction during the addition reaction with hydrogensiloxane. Therefore, it is possible to manufacture a polysiloxane compound with higher purity than an unsaturated group-containing compound having a primary hydroxyl group or a secondary hydroxyl group. Furthermore, the target product can be produced even at a high modification rate. In addition, because the product can be synthesized with high purity even when the mole ratio of the unsaturated compound to hydrogensiloxy units is low, the amount of the unsaturated compound to be used can be reduced, making it excellent in terms of yield and cost efficiency.

[Examples 2-1 to 2-8 and Comparative Examples 2-1 to 2-3]

Example of Manufacturing Hydrogel

Each compound obtained in Examples 1-1 to 1-8, each compound obtained in Comparative Synthesis Example 1-1 to 1-2, and 1-5, 2-hydroxyethyl methacrylate (HEMA), N,N-dimethylacrylamide (DMA), ethylene glycol dimethacrylate (EGDMA), Irgacure 1173 (IRG1173), and Isopropanol (IPA) were mixed according to the composition and blending ratios shown in Table 1, and stirred until being homogeneous. After stirring, the mixture was subjected to bubbling with $N_2$ for 5 minutes, deaerated sufficiently, and then encapsulated in a polypropylene mold. The mixture was cured by UV irradiation using a high-pressure mercury lamp. After being cured, the cured product was cleaned by being immersed in isopropanol, a 50% isopropanol aqueous solution, and deionized water, in this order for a few seconds, respectively, to obtain a hydrogel film. Each physical property of the obtained films was measured according to the method described below. The results are shown in Table 1.

[Appearance after Curing]

The appearance after curing was visually observed and evaluated according to the following criteria.

A: Uniform and clear
B: Non-uniform or cloudy

[Transparency]

After the film was immersed in deionized water at 25°C for 48 hours, the water on the surface was wiped off to produce a hydrated film. The appearance was visually observed and evaluated according to the following criteria.

A: Uniform and clear
B: Non-uniform or cloudy

[Equilibrium Moisture Content]

After the film was immersed in deionized water at 25°C for 48 hours, the water on the surface was wiped off, and the mass of the hydrated film was measured. Next, the hydrated film was dried in an oven at 50°C for 48 hours and in an oven at 25°C for 24 hours, and then the mass of the dried film was measured. Equilibrium moisture content was calculated using the following formula.

Equilibrium moisture content (%) = 100 x (mass of hydrated film - mass of dried film) / mass of hydrated film

[Modulus of Elasticity (Young's elasticity modulus) and Elongation Rate]

After the film was immersed in deionized water at 25°C for 48 hours, the water on the surface was wiped off to produce a hydrated film. The Young's elasticity modulus of the hydrated film was measured using an Instron 5943. A film cut to 0.8 cm x 4.0 cm was measured with a 50 N load cell at a head speed of 1 cm/min. The slope of the initial part (straight-line segment) of a stress-strain curve, obtained when stress is plotted on the vertical axis and strain on the horizontal axis, was calculated and defined as Young's elasticity modulus (MPa). The elongation rate when the film was broken was defined as the maximum elongation rate (%).

Table 1

| | | Example 2-1 | Example 2-2 | Example 2-3 | Example 2-4 | Example 2-5 | Example 2-6 | Example 2-7 | Example 2-8 | Comparative Example 2-1 | Comparative Example 2-2 | Comparative Example 2-3 |
|---|---|---|---|---|---|---|---|---|---|---|---|---|
| Polysiloxane compound | 100A | 50 | 0 | 0 | 0 | 0 | 0 | 0 | 25 | 0 | 0 | 0 |
| | 100C | 0 | 50 | 0 | 0 | 0 | 0 | 0 | 0 | 0 | 0 | 0 |
| | 101A | 0 | 0 | 50 | 0 | 0 | 0 | 0 | 0 | 0 | 0 | 0 |
| | 102A | 0 | 0 | 0 | 50 | 0 | 0 | 0 | 0 | 0 | 0 | 0 |
| | 102B | 0 | 0 | 0 | 0 | 50 | 0 | 0 | 0 | 0 | 0 | 0 |
| | 103A | 0 | 0 | 0 | 0 | 0 | 50 | 0 | 0 | 0 | 0 | 0 |
| | 103C | 0 | 0 | 0 | 0 | 0 | 0 | 50 | 0 | 0 | 0 | 0 |
| | 104A | 0 | 0 | 0 | 0 | 0 | 0 | 0 | 25 | 0 | 0 | 0 |
| Comparative polysiloxane compound | 200 | 0 | 0 | 0 | 0 | 0 | 0 | 0 | 0 | 50 | 0 | 0 |
| | 201 | 0 | 0 | 0 | 0 | 0 | 0 | 0 | 0 | 0 | 50 | 0 |
| | 200P | 0 | 0 | 0 | 0 | 0 | 0 | 0 | 0 | 0 | 0 | 50 |
| Hydrophilic monomer | HEMA | 25 | 25 | 25 | 25 | 25 | 25 | 25 | 25 | 25 | 25 | 25 |
| | DMA | 25 | 25 | 25 | 25 | 25 | 25 | 25 | 25 | 25 | 25 | 25 |
| | EGDMA | 1 | 1 | 1.7 | 1.1 | 1.1 | 1.2 | 1.2 | 1.5 | 1 | 1 | 1.7 |
| Initiator | IRG11 73 | 1 | 1 | 1 | 1 | 1 | 1 | 1 | 1 | 1 | 1 | 1 |
| Solvent | IPA | 30 | 30 | 30 | 30 | 30 | 30 | 30 | 30 | 30 | 30 | 30 |
| Evaluation result | Appearance after curing | A | A | A | A | A | A | A | A | B | B | A |
| | Transparency | A | A | A | A | A | A | A | A | B | B | B |
| | Equilibrium moisture content (%) | 53 | 54 | 55 | 65 | 63 | 57 | 57 | 59 | 48 | 52 | 56 |
| | Modulus of elasticity (MPa) | 1.2 | 1.1 | 0.9 | 1.4 | 1.4 | 1.3 | 1.4 | 1 | 2.3 | 2.1 | 2.5 |
| | Maximum elongation rate (%) | 70 | 70 | 110 | 80 | 100 | 80 | 90 | 70 | 10 | 10 | 40 |

As shown in Table 1, the hydrogels of Examples 2-1 to 2-8, using the inventive polysiloxane compound, are excellent in transparency, modulus of elasticity, and maximum elongation rate. On the other hand, as shown in Table 1, the hydrogels of Comparative Examples 2-1 to 2-3, which used compounds (200), (201), or (200P), each having a primary hydroxyl group or a secondary hydroxyl group as a comparative compound, had a problem in transparency after curing or after hydration, and in addition, had a high elastic modulus and a low maximum elongation. That is, the hydrogel using the inventive polysiloxane compound provides a medical material having beneficial hydrophilicity, sufficient strength, and sufficient flexibility.

[INDUSTRIAL APPLICABILITY]

The inventive polysiloxane compound provides a hydrogel having excellent hydrophilicity and excellent strength. The inventive polysiloxane compounds are useful as monomers for manufacturing medical materials, such as ophthalmic devices, contact lenses, intraocular lenses, artificial corneas, and spectacle lenses.
The present description includes the following embodiments.

[1]: A polysiloxane compound comprising one or more (meth)acryloxy groups per molecule, wherein the polysiloxane compound has a tertiary hydroxyl group at the terminal of a side chain.
[2]: The polysiloxane compound of the above [1], wherein the polysiloxane compound is represented by the following general formula (1),

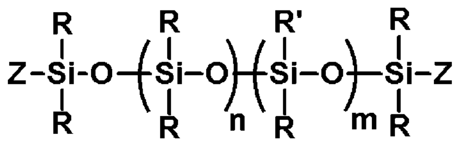

(1)

wherein "Z" each independently represents an organic group having 1 to 20 carbon atoms; "R" each independently represents a monovalent hydrocarbon group having 1 to 6 carbon atoms; "R'" each independently represents a monovalent hydrocarbon group having a tertiary hydroxyl group at the terminal, having 3 to 20 carbon atoms, and optionally having an ether bond at the non-terminal of the group; "n" represents an integer of 0 to 200; "m" represents an integer of 3 to 200; and one or more of "Z"s are a (meth)acryloxy group.
[3]: The polysiloxane compound of the above [2], wherein, in the general formula (1), "Z" is represented by the following general formula (2),

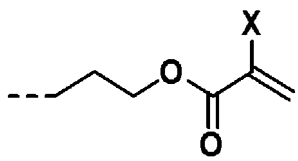

(2)

wherein "X" represents a hydrogen atom or a methyl group, and a broken line represents an attachment point to a silicon atom.
[4]: The polysiloxane compound of the above [2] or [3], wherein, in the general formula (1), "R" represents a methyl group.
[5]: The polysiloxane compound of any one of the above [2] to [4], wherein, in the general formula (1), "R'" is represented by the following general formula (3) or (4),

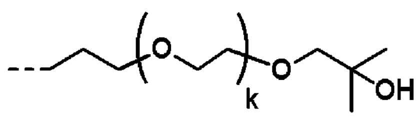

(3)

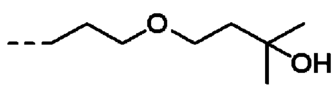

(4)

wherein "k", in the general formula (3), represents an integer of 0 to 5, and a broken line, in the general formulae (3) and (4), represents an attachment point to a silicon atom.
[6]: The polysiloxane compound of the above [5], wherein, in the general formula (3), "k" represents 0 or 1.

[7]: The polysiloxane compound of any one of the above [2] to [6], wherein, in the general formula (1), "n" represents an integer of 0 to 100, "m" represents an integer of 3 to 100, and m/(n+m) is 0.15 or more.
[8]: The polysiloxane compound of the above [7], wherein, in the general formula (1), m/(n+m) is 0.3 or more.
[9]: A polymer being a polymerized product of the polysiloxane compound of any one of the above [1] to [8].
[10]: The polymer of the above [9], wherein the mass ratio of the polysiloxane compound is 20 mass% or more relative to the total mass of the polymer.
[11]: A hydrogel comprising the polymer of the above [9] or [10].
[12]: A medical material comprising the polymer of the above [9] or [10].
[13]: A method for manufacturing a polysiloxane compound,

wherein the method for manufacturing the polysiloxane compound comprises a step of obtaining the polysiloxane compound represented by the general formula (1) by a hydrosilylation of a compound having an unsaturated group and a tertiary hydroxyl group at the terminal, and an organohydrogenpolysiloxane compound represented by the following general formula (5),

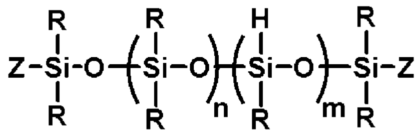

(5)

wherein "Z" each independently represents an organic group having 1 to 20 carbon atoms; "R" each independently represents a monovalent hydrocarbon group having 1 to 6 carbon atoms; "n" represents an integer of 0 to 200; "m" represents an integer of 3 to 200; and one or more of "Z"s are a (meth)acryloxy group,

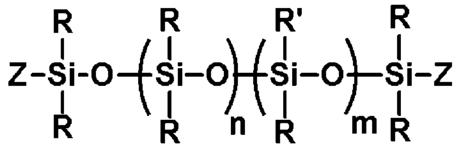

(1)

wherein "R'" each independently represents a monovalent hydrocarbon group having a tertiary hydroxyl group at the terminal, having 3 to 20 carbon atoms, and optionally having an ether bond at the non-terminal of the group; and "Z", "R", "n", and "m" represent the same defined above.

[14]: The method for manufacturing a polysiloxane compound of the above [13], wherein the compound having an unsaturated group and a tertiary hydroxyl group at the terminal is contained in 1.0 to 3.0 molar equivalents relative to a hydrogensiloxy unit.
[15]: The method for manufacturing a polysiloxane compound of the above [13] or [14], wherein the content ratio of the polysiloxane compound represented by the general formula (1) is 90% or more.

It should be noted that the present invention is not limited to the above-described embodiments. The above embodiments are just examples, and any examples that have substantially the same feature and demonstrate the same functions and effects as those in the technical concept disclosed in claims of the present invention are included in the technical scope of the present invention.

## Claims

1. A polysiloxane compound comprising one or more (meth)acryloxy groups per molecule, wherein the polysiloxane compound has a tertiary hydroxyl group at the terminal of a side chain.

2. The polysiloxane compound according to claim 1, wherein the polysiloxane compound is represented by the following general formula (1),

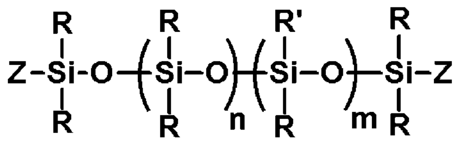

(1)

wherein "Z" each independently represents an organic group having 1 to 20 carbon atoms; "R" each independently represents a monovalent hydrocarbon group having 1 to 6 carbon atoms; "R'" each independently represents a monovalent hydrocarbon group having a tertiary hydroxyl group at the terminal, having 3 to 20 carbon atoms, and optionally having an ether bond at the non-terminal of the group; "n" represents an integer of 0 to 200; "m" represents an integer of 3 to 200; and one or more of "Z"s are a (meth)acryloxy group.

3. The polysiloxane compound according to claim 2, wherein, in the general formula (1), "Z" is represented by the following general formula (2),

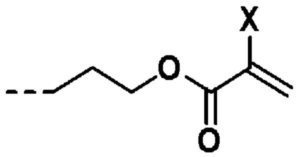

(2)

wherein "X" represents a hydrogen atom or a methyl group, and a broken line represents an attachment point to a silicon atom.

4. The polysiloxane compound according to claim 2, wherein, in the general formula (1), "R" represents a methyl group.

5. The polysiloxane compound according to claim 2, wherein, in the general formula (1), "R'" is represented by the following general formula (3) or (4),

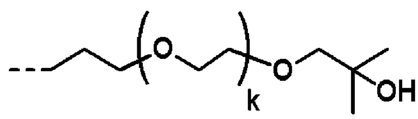

(3)

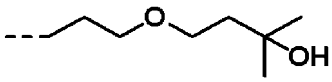

(4)

wherein "k", in the general formula (3), represents an integer of 0 to 5, and a broken line, in the general formulae (3) and (4), represents an attachment point to a silicon atom.

6. The polysiloxane compound according to claim 5, wherein, in the general formula (3), "k" represents 0 or 1.

7. The polysiloxane compound according to claim 2, wherein, in the general formula (1), "n" represents an integer of 0 to 100, "m" represents an integer of 3 to 100, and m/(n+m) is 0.15 or more.

8. The polysiloxane compound according to claim 7, wherein, in the general formula (1), m/(n+m) is 0.3 or more.

9. A polymer being a polymerized product of the polysiloxane compound according to any one of claims 1 to 8.

10. The polymer according to claim 9, wherein the mass ratio of the polysiloxane compound is 20 mass% or more relative to the total mass of the polymer.

11. A hydrogel comprising the polymer according to claim 9.

12. A medical material comprising the polymer according to claim 9.

13. A method for manufacturing a polysiloxane compound,

wherein the method for manufacturing the polysiloxane compound comprises a step of obtaining the polysiloxane compound represented by the general formula (1) by a hydrosilylation of a compound having an unsaturated group and a tertiary hydroxyl group at the terminal, and an organohydrogenpolysiloxane compound represented by the following general formula (5),

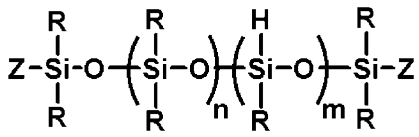

(5)

wherein "Z" each independently represents an organic group having 1 to 20 carbon atoms; "R" each independently represents a monovalent hydrocarbon group having 1 to 6 carbon atoms; "n" represents an integer of 0 to 200; "m" represents an integer of 3 to 200; and one or more of "Z"s are a (meth)acryloxy group,

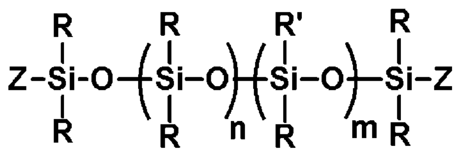

(1)

wherein "R'" each independently represents a monovalent hydrocarbon group having a tertiary hydroxyl group at the terminal, having 3 to 20 carbon atoms, and optionally having an ether bond at the non-terminal of the group; and "Z", "R", "n", and "m" represent the same defined above.

**14.** The method for manufacturing a polysiloxane compound according to claim 13, wherein the compound having an unsaturated group and a tertiary hydroxyl group at the terminal is contained in 1.0 to 3.0 molar equivalents relative to a hydrogensiloxy unit.

**15.** The method for manufacturing a polysiloxane compound according to claim 13 or 14, wherein the content ratio of the polysiloxane compound represented by the general formula (1) is 90% or more.

**INTERNATIONAL SEARCH REPORT**

International application No.
**PCT/JP2024/020976**

**A. CLASSIFICATION OF SUBJECT MATTER**

***C08G 77/38***(2006.01)i; ***A61L 27/00***(2006.01)i; ***A61L 27/18***(2006.01)i; ***C08F 290/06***(2006.01)i
FI: C08G77/38; C08F290/06; A61L27/18; A61L27/00

According to International Patent Classification (IPC) or to both national classification and IPC

**B. FIELDS SEARCHED**

Minimum documentation searched (classification system followed by classification symbols)

C08G77/38; A61L27/00; A61L27/18; C08F290/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2020/246313 A1 (SHIN-ETSU CHEMICAL CO., LTD.) 10 December 2020 (2020-12-10)<br>claims, examples | 1-15 |
| A | JP 2023-518030 A (ALCON INC.) 27 April 2023 (2023-04-27)<br>claims, examples | 1-15 |
| A | JP 2018-090735 A (SHIN-ETSU CHEMICAL CO., LTD.) 14 June 2018 (2018-06-14)<br>claims, examples | 1-15 |
| A | JP 2019-143060 A (SHIN-ETSU CHEMICAL CO., LTD.) 29 August 2019 (2019-08-29)<br>claims, examples | 1-15 |
| A | JP 2021-088689 A (SHIN-ETSU CHEMICAL CO., LTD.) 10 June 2021 (2021-06-10)<br>claims, examples | 1-15 |

☐ Further documents are listed in the continuation of Box C.

☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 August 2024** | **13 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/020976**

| Patent document cited in search report | Publication date (day/month/year) | Patent family member(s) | Publication date (day/month/year) |
|---|---|---|---|
| WO 2020/246313 A1 | 10 December 2020 | US 2022/0332863 A1<br>claims, examples<br>EP 3981802 A1<br>CN 113906037 A | |
| JP 2023-518030 A | 27 April 2023 | US 2021/0292558 A1<br>claims, examples<br>WO 2021/186383 A1<br>EP 4121803 A1<br>CN 115298574 A<br>KR 10-2022-0140859 A | |
| JP 2018-090735 A | 14 June 2018 | (Family: none) | |
| JP 2019-143060 A | 29 August 2019 | US 2020/0385530 A1<br>claims, examples<br>WO 2019/163668 A1<br>EP 3757151 A1<br>CN 111757905 A<br>KR 10-2020-0123440 A | |
| JP 2021-088689 A | 10 June 2021 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005170827 A
- JP 2021073346 A